# EUROPEAN PATENT SPECIFICATION

(11) **EP 2 359 142 B1**
(45) Date of publication and mention of the grant of the patent: **03.07.2013**
(21) Application number: 09744181.0
(22) Date of filing: 08.10.2009
(51) Int. Cl.: G01N 33/68

(54) **Importin 9 as biomarker for schizophrenia**
Importin 9 als Biomarker der Schizophrenie
Importin 9 en tant que biomarqueur de la schizophrénie

(30) Priority: 10.10.2008 GB 0818660
(43) Date of publication of application: 24.08.2011
(73) Proprietor: Cambridge Enterprise Limited, Trinity Lane Cambridge Cambridgeshire CB2 1TN (GB)
(72) Inventor: LEVIN, Yishai, Hadera 38523 (IL); WANG, Lan, Bristol BS32 8BP (GB); BAHN, Sabine, Cambridgeshire CB2 1QT (GB)
(74) Representative: Gibson, Mark
(86) International application number: PCT/GB2009/051336
(87) International publication number: WO 2010/041069

(56) References cited:
- WO-A1-2008/107700
- WO-A2-2008/090319
- US-A1- 2007 224 644
- KORTVELY E ET AL: "Cloning and characterization of rat importin 9: Implication for its neuronal function" MOLECULAR BRAIN RESEARCH, ELSEVIER SCIENCE BV, AMSTERDAM, NL, vol. 139, no. 1, 13 September 2005 (2005-09-13), pages 103-114, XP025297265 ISSN: 0169-328X [retrieved on 2005-09-13]
- PERSSON ET AL: "Characterization of the 12q amplicons by high-resolution, oligonucleotide array CGH and expression analyses of a novel liposarcoma cell line" CANCER LETTERS, NEW YORK, NY, US, vol. 260, no. 1-2, 21 December 2007 (2007-12-21), pages 37-47, XP022424274 ISSN: 0304-3835

## Description

### Field of the invention

The present invention relates to a method of diagnosing or monitoring schizophrenia or a predisposition thereto.

### Background of the Invention

Schizophrenia is a multifaceted neuropsychiatric disease with an onset and aetiology driven by a complex interplay of genetic, developmental, nutritional and environmental factors. It affects at least 1% of the world population and costs hundreds of billions of U.S. dollars in healthcare provision and lost earnings.

Current diagnosis of schizophrenia and other psychotic disorders is subjective due to the wide spectrum of symptoms, their overlap with other disorders and the lack of empirical disease-specific assays. An increased understanding of the affected molecular pathways would be an important step toward improving diagnostic tests, developing new pre-symptomatic treatments and assessing the efficacy of preventative interventions.

WO 2008/090319 describes a method of diagnosing or monitoring a psychotic disorder, or predisposition thereto, comprising measuring, in a sample taken from a subject, the level of a biomarker selected from clusterin precursor, inter a-trypsin inhibitor, IgM, apolipoprotein A2 and a2 H5 glycoprotein. WO 2008/107700 describes the use of a peptide biomarker which is an α-defensin or a fragment thereof, as a biomarker for schizophrenia or a major depressive disorder, or predisposition thereto.

### Summary of the Invention

Using a global proteomic platform based on 1 dimensional and 2 dimensional nanoLC-MS/MS technology protein disease biomarkers for schizophrenia, have been identified. Two discovery studies were conducted, using serum samples taken from first-onset, drug-naïve schizophrenia patients and healthy controls.

According to a first aspect, the present invention is the *in vitro* use of Importin 9 as a biomarker for schizophrenia, or predisposition thereto. In one embodiment, the invention additionally comprises the use of one or more additional peptides selected from, NALP8, Vitamin K-dependent protein S, Alpha-1-antichymotrypsin, Lumican, Helicase, Haptoglobin related, ANKRD26-like family C member 1B, ATPase family AAA domain-containing protein 2B, Uncharacterized protein C9orf93, Thyroxine-binding globulin, EF-hand domain-containing family member A1, Alpha-aminoadipic semialdehyde synthase, mitochondrial, 1-phosphatidylinositol-4,5-bisphosphate phosphodiesterase gamma-1, Cytosolic carboxypeptidase 2, Alpha-1-antichymotrypsin, Uncharacterized protein FLJ36157, Attractin, Rootletin, Complement component 7, Afamin, Kinesin-like protein, Poly [ADP-ribose] polymerase 1 (PARP1), Ig gamma-1 chain C region, Serum paraoxonase/arylesterase 1, Inter-alpha-trypsin inhibitor heavy chain H2, Inter-alpha-trypsin inhibitor heavy chain H4, Apolipoprotein C-III, Pregnancy Zone protein, Heparin cofactor 2, Complement C1r subcomponent, as a biomarker for schizophrenia, or predisposition thereto.

In one embodiment, the invention additionally comprises the use of one or more additional peptides selected from E3 ubiquitin-protein ligase LRSAM1, IgMu, Coagulation factor 13, Apolipoprotein C1, Apolipoprotein D, Serine/threonine-protein kinase, Mitochondrial ATP-binding cassette sub-family B member 6, E3 ubiquitin-protein ligase, Haptoglobin, ATPase family AAA domain-containing protein 2, Spectrin beta chain erythrocyte, Cartilage oligomeric matrix protein, Mannose-binding protein C, Integrin beta-3, Microtubule-associated protein 2, Cathepsin L2, Complement factor B, Complement component 5, Vitronectin; S-protein, Clusterin (ApoJ), Hyaluronan-binding protein 2 and DNA topoisomerase 2-alpha, as a biomarker for schizophrenia, or predisposition thereto

According to a further aspect, the present invention is a method of diagnosing or monitoring schizophrenia, or predisposition thereto, comprising detecting and/or quantifying, in a sample from a test subject , one or more of the peptide biomarkers listed above.

According to a further aspect, the present invention is a method of monitoring efficacy of a therapy in a subject having, suspected of having, or of being predisposed to schizophrenia, comprising detecting and/or quantifying, in a sample from said subject, one or more of the peptide biomarkers listed above.

A further aspect of the invention provides ligands, such as naturally occurring or chemically synthesised compounds, capable of specific binding to the peptide biomarker. A ligand according to the invention may comprise a peptide, an antibody or a fragment thereof, or an aptamer or oligonucleotide, capable of specific binding to the peptide biomarker. The antibody can be a monoclonal antibody or a fragment thereof capable of specific binding to the peptide biomarker. A ligand according to the invention may be labelled with a detectable marker, such as a luminescent, fluorescent or radioactive marker; alternatively or additionally a ligand according to the invention may be labelled with an affinity tag, e.g. a biotin, avidin, streptavidin or His (e.g. hexa-His) tag.

A biosensor according to the invention may comprise the peptide biomarker or a structural/shape mimic thereof capable of specific binding to an antibody against the peptide biomarker. Also provided is an array comprising a ligand or mimic as described herein.

Also provided by the invention is the use of one or more ligands as described herein, which may be naturally occurring or chemically synthesised, and is suitably a peptide, antibody or fragment thereof, aptamer or oligonucleotide, or the use of a biosensor of the invention, or an array of the invention, or a kit of the invention to detect and/or quantify the peptide. In these uses, the detection and/or quantification can be performed on a biological sample such as from the group consisting of CSF, whole blood, blood serum, plasma, urine, saliva, or other bodily fluid, breath, e.g. as condensed breath, or an extract or purification therefrom, or dilution thereof.

Diagnostic or monitoring kits are provided for performing methods of the invention. Such kits will suitably comprise a ligand according to the invention, for detection and/or quantification of the peptide biomarker, and/or a biosensor, and/or an array as described herein, optionally together with instructions for use of the kit.

A further aspect of the invention is the use of a kit for monitoring or diagnosing schizophrenia, wherein said kit comprises a biosensor which detects and/or quantifies the biomarker as described herein, wherein said biosensor comprises a ligand capable of specific binding to the biomarker as described herein.

Biomarkers for schizophrenia or other psychotic disorders are essential targets for discovery of novel targets and drug molecules that retard or halt progression of the disorder. As the level of the peptide biomarker is indicative of disorder and of drug response, the biomarker is useful for identification of novel therapeutic compounds in *in vitro* and/or *in vivo* assays. Biomarkers of the invention can be employed in methods for screening for compounds that modulate the activity of the peptide.

Thus, in a further aspect of the invention, there is provided the use of a ligand, as described, which can be a peptide, antibody or fragment thereof or aptamer or oligonucleotide according to the invention; or the use of a biosensor according to the invention, or an array according to the invention; or a kit according to the invention, to identify a substance capable of promoting and/or of suppressing the generation of the biomarker.

Also there is provided a method of identifying a substance capable of promoting or suppressing the generation of the peptide in a subject, comprising administering a test substance to a subject animal and detecting and/or quantifying the level of the peptide biomarker present in a test sample from the subject.

### Description of the Invention

The term "biomarker" means a distinctive biological or biologically derived indicator of a process, event, or condition. Peptide biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment. The peptide biomarkers described herein may also be in 'precursor' form.

References herein to "other psychotic disorder" relate to any appropriate psychotic disorder according to DSM-IV Diagnostic and Statistical Manual of Mental Disorders, 4th edition, American Psychiatric Assoc, Washington, D.C., 2000. In one particular embodiment, the other psychotic disorder is a psychotic disorder related to schizophrenia. Examples of psychotic disorders related to schizophrenia include brief psychotic disorder delusional disorder, psychotic disorder due to a general medical condition, schizoeffective disorder, schizophreniform disorder, and substance-induced psychotic disorder.

As used herein, the term "biosensor" means anything capable of detecting the presence of the biomarker. Examples of biosensors are described herein.

Biosensors according to the invention may comprise a ligand or ligands, as described herein, capable of specific binding to the peptide biomarker. Such biosensors are useful in detecting and/or quantifying a peptide of the invention.

Diagnostic kits for the diagnosis and monitoring of schizophrenia are described herein. Preferably, they contain a biosensor capable of detecting and/or quantifying a peptide biomarker.

Monitoring methods of the invention can be used to monitor onset, progression, stabilisation, amelioration and/or remission.

In methods of diagnosing or monitoring according to the invention, detecting and/or quantifying the peptide biomarker in a biological sample from a test subject may be performed on two or more occasions. Comparisons may be made between the level of biomarker in samples taken on two or more occasions. Assessment of any change in the level of the peptide biomarker in samples taken on two or more occasions may be performed. Modulation of the peptide biomarker level is useful as an indicator of the state of the schizophrenia or predisposition thereto. An increase in the level of the biomarker, over time is indicative of onset or progression, i.e. worsening of this disorder, whereas a decrease in the level of the peptide biomarker indicates amelioration or remission of the disorder, or vice versa.

A method of diagnosis of or monitoring according to the invention may comprise quantifying the peptide biomarker in a test biological sample from a test subject and comparing the level of the peptide present in said test sample with one or more controls.

The control used in a method of the invention can be one or more control(s) selected from the group consisting of: the level of biomarker peptide found in a normal control sample from a normal subject, a normal biomarker peptide level; a normal biomarker peptide range, the level in a sample from a subject with schizophrenia or another psychotic disorder, or a diagnosed predisposition thereto; schizophrenia or another psychotic disorder biomarker peptide level, or schizophrenia or another psychotic disorder biomarker peptide range.

A preferred method of diagnosing schizophrenia, or predisposition thereto, comprises:
(a) quantifying the amount of the peptide biomarker in a test biological sample; and
(b) comparing the amount of said peptide in said test sample with the amount present in a normal control biological sample from a normal subject.

A higher level of the peptide biomarker in the test sample relative to the level in the normal control is indicative of the presence of schizophrenia, or predisposition thereto; an equivalent or lower level of the peptide in the test sample relative to the normal control is indicative of absence of schizophrenia and/or absence of a predisposition thereto.

The term "diagnosis" as used herein encompasses identification, confirmation, and/or characterisation of schizophrenia, or predisposition thereto. By predisposition it is meant that a subject does not currently present with the disorder, but is liable to be affected by the disorder in time. Methods of monitoring and of diagnosis according to the invention are useful to confirm the existence of a disorder, or predisposition thereto; to monitor development of the disorder by assessing onset and progression, or to assess amelioration or regression of the disorder. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing "down-time" and relapse rates.

Also provided is a method of monitoring efficacy of a therapy for schizophrenia in a subject having such a disorder, suspected of having such a disorder, or of being predisposed thereto, comprising detecting and/or quantifying the peptide present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the level of the biomarker(s) in test samples taken on different occasions.

The invention provides a method for monitoring efficacy of therapy for schizophrenia in a subject, comprising:
(a) quantifying the amount of the peptide biomarker; and
(b) comparing the amount of said peptide in said test sample with the amount present in one or more control(s) and/or one or more previous test sample(s) taken at an earlier time from the same test subject.

A decrease in the level of the peptide biomarker in the test sample relative to the level in a previous test sample taken earlier from the same test subject is indicative of a beneficial effect, e.g. stabilisation or improvement, of said therapy on the disorder, suspected disorder or predisposition thereto.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an anti-depressant therapy. Samples can be taken at intervals over the remaining life, or a part thereof, of a subject.

The term "detecting" as used herein means confirming the presence of the peptide biomarker present in the sample. Quantifying the amount of the biomarker present in a sample may include determining the concentration of the peptide biomarker present in the sample. Detecting and/or quantifying may be performed directly on the sample, or indirectly on an extract therefrom, or on a dilution thereof.

In alternative aspects of the invention, the presence of the peptide biomarker is assessed by detecting and/or quantifying antibody or fragments thereof capable of specific binding to the biomarker that are generated by the subject's body in response to the peptide and thus are present in a biological sample from a subject having schizophrenia or a predisposition thereto.

Detecting and/or quantifying can be performed by any method suitable to identify the presence and/or amount of a specific protein in a biological sample from a patient or a purification or extract of a biological sample or a dilution thereof. In methods of the invention, quantifying may be performed by measuring the concentration of the peptide biomarker in the sample or samples. Biological samples that may be tested in a method of the invention include cerebrospinal fluid (CSF), whole blood, blood serum, plasma, urine, saliva, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken post-mortem. The samples can be prepared, for example where appropriate diluted or concentrated, and stored in the usual manner.

Detection and/or quantification of peptide biomarkers may be performed by detection of the peptide biomarker or of a fragment thereof, e.g. a fragment with C-terminal truncation, or with N-terminal truncation. Fragments are suitably greater than 4 amino acids in length, preferably 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring according to the invention may comprise analysing a sample of cerebrospinal fluid (CSF) by SELDI TOF or MALDI TOF to detect the presence or level of the peptide biomarker. These methods are also suitable for clinical screening, prognosis, monitoring the results of therapy, identifying patients most likely to respond to a particular therapeutic treatment, for drug screening and development, and identification of new targets for drug treatment.

Detecting and/or quantifying the peptide biomarkers may be performed using an immunological method, involving an antibody, or a fragment thereof capable of specific binding to the peptide biomarker. Suitable immunological methods include sandwich immunoassays, such as sandwich ELISA, in which the detection of the peptide biomarkers is performed using two antibodies which recognize different epitopes on a peptide biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

Immunological methods in accordance with the invention may be based, for example, on any of the following methods.

Immunoprecipitation is the simplest immunoassay method; this measures the quantity of precipitate, which forms after the reagent antibody has incubated with the sample and reacted with the target antigen present therein to form an insoluble aggregate. Immunoprecipitation reactions may be qualitative or quantitative.

In particle immunoassays, several antibodies are linked to the particle, and the particle is able to bind many antigen molecules simultaneously. This greatly accelerates the speed of the visible reaction. This allows rapid and sensitive detection of the biomarker.

In immunonephelometry, the interaction of an antibody and target antigen on the biomarker results in the formation of immune complexes that are too small to precipitate. However, these complexes will scatter incident light and this can be measured using a nephelometer. The antigen, i.e. biomarker, concentration can be determined within minutes of the reaction.

Radioimmunoassay (RIA) methods employ radioactive isotopes such as I¹²⁵ to label either the antigen or antibody. The isotope used emits gamma rays, which are usually measured following removal of unbound (free) radiolabel. The major advantages of RIA, compared with other immunoassays, are higher sensitivity, easy signal detection, and well-established, rapid assays. The major disadvantages are the health and safety risks posed by the use of radiation and the time and expense associated with maintaining a licensed radiation safety and disposal program. For this reason, RIA has been largely replaced in routine clinical laboratory practice by enzyme immunoassays.

Enzyme (EIA) immunoassays were developed as an alternative to radioimmunoassays (RIA). These methods use an enzyme to label either the antibody or target antigen. The sensitivity of EIA approaches that for RIA, without the danger posed by radioactive isotopes. One of the most widely used EIA methods for detection is the enzyme-linked immunosorbent assay (ELISA). ELISA methods may use two antibodies one of which is specific for the target antigen and the other of which is coupled to an enzyme, addition of the substrate for the enzyme results in production of a chemoluminescent or fluorescent signal.

Fluorescent immunoassay (FIA) refers to immunoassays which utilize a fluorescent label or an enzyme label which acts on the substrate to form a fluorescent product. Fluorescent measurements are inherently more sensitive than colorimetric (spectrophotometric) measurements. Therefore, FIA methods have greater analytical sensitivity than EIA methods, which employ absorbance (optical density) measurement.

Chemiluminescent immunoassays utilize a chemiluminescent label, which produces light when excited by chemical energy; the emissions are measured using a light detector.

Immunological methods according to the invention can thus be performed using well-known methods. Any direct (e.g., using a sensor chip) or indirect procedure may be used in the detection of peptide biomarkers of the invention.

The Biotin-Avidin or Biotin-Streptavidin systems are generic labelling systems that can be adapted for use in immunological methods of the invention. One binding partner (hapten, antigen, ligand, aptamer, antibody, enzyme etc) is labelled with biotin and the other partner (surface, e.g. well, bead, sensor etc) is labelled with avidin or streptavidin. This is conventional technology for immunoassays, gene probe assays and (bio)sensors, but is an indirect immobilisation route rather than a direct one. For example a biotinylated ligand (e.g. antibody or aptamer) specific for a peptide biomarker of the invention may be immobilised on an avidin or streptavidin surface, the immobilised ligand may then be exposed to a sample containing or suspected of containing the peptide biomarker in order to detect and/or quantify a peptide biomarker of the invention. Detection and/or quantification of the immobilised antigen may then be performed by an immunological method as described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, i.e., molecules that contain an antigen binding site that specifically binds an antigen. The immunoglobulin molecules of the invention can be of any class (e. g., IgG, IgE, IgM, IgD and IgA) or subclass of immunoglobulin molecule.

The identification of key biomarkers specific to a disease is central to integration of diagnostic procedures and therapeutic regimes. Using predictive biomarkers appropriate diagnostic tools such as biosensors can be developed, accordingly, in methods and uses of the invention, detecting and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

Thus, according to a further aspect of the invention there is provided an apparatus for diagnosing or monitoring schizophrenia which comprises a biosensor, microanalytical, microengineered, microseparation and/or immunochromatography system configured to detect and/or quantify any of the biomarkers defined herein.

The biomarker(s) of the invention can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, e.g. in the ward, outpatients' department, surgery, home, field and workplace.

Biosensors to detect one or more biomarkers of the invention include acoustic, plasmon resonance, holographic and microengineered sensors. Imprinted recognition elements, thin film transistor technology, magnetic acoustic resonator devices and other novel acousto-electrical systems may be employed in biosensors for detection of the one or more biomarkers of the invention.

Methods involving detection and/or quantification of one or more peptide biomarkers of the invention can be performed on bench-top instruments, or can be incorporated onto disposable, diagnostic or monitoring platforms that can be used in a non-laboratory environment, e.g. in the physician's office or at the patient's bedside. Suitable biosensors for performing methods of the invention include "credit" cards with optical or acoustic readers. Biosensors can be configured to allow the data collected to be electronically transmitted to the physician for interpretation and thus can form the basis for e-neuromedicine.

Any suitable animal may be used as a subject non-human animal, for example a non-human primate, horse, cow, pig, goat, sheep, dog, cat, fish, rodent, e.g. guinea pig, rat or mouse; insect (e.g. *Drosophila*), amphibian (e.g. *Xenopus*) or *C*. *elegans.*

The test substance can be a known chemical or pharmaceutical substance, such as, but not limited to, an anti-depressive disorder therapeutic; or the test substance can be a novel synthetic or natural chemical entity, or a combination of two or more of the aforesaid substances.

There is provided a method of identifying a substance capable of promoting or suppressing the generation of the peptide biomarker in a subject, comprising exposing a test cell to a test substance and monitoring the level of the peptide biomarker within said test cell, or secreted by said test cell.

The test cell could be prokaryotic, however it is preferred that a eukaryotic cell be employed in cell-based testing methods. Suitably, the eukaryotic cell is a yeast cell, insect cell, *Drosophila* cell, amphibian cell (e.g. from *Xenopus*), *C. elegans* cell or is a cell of human, non-human primate, equine, bovine, porcine, caprine, ovine, canine, feline, piscine, rodent or murine origin.

In methods for identifying substances of potential therapeutic use, non-human animals or cells can be used that are capable of expressing the peptide.

Screening methods also encompass a method of identifying a ligand capable of binding to the peptide biomarker according to the invention, comprising incubating a test substance in the presence of the peptide biomarker in conditions appropriate for binding, and detecting and/or quantifying binding of the peptide to said test substance.

High-throughput screening technologies based on the biomarker, uses and methods of the invention, e.g. configured in an array format, are suitable to monitor biomarker signatures for the identification of potentially useful therapeutic compounds, e.g. ligands such as natural compounds, synthetic chemical compounds (e.g. from combinatorial libraries), peptides, monoclonal or polyclonal antibodies or fragments thereof, which may be capable of binding the biomarker.

Methods of the invention can be performed in array format, e.g. on a chip, or as a multiwell array. Methods can be adapted into platforms for single tests, or multiple identical or multiple non-identical tests, and can be performed in high throughput format. Methods of the invention may comprise performing one or more additional, different tests to confirm or exclude diagnosis, and/or to further characterise a condition.

The invention further provides a substance, e.g. a ligand, identified or identifiable by an identification or screening method or use of the invention. Such substances may be capable of inhibiting, directly or indirectly, the activity of the peptide biomarker, or of suppressing generation of the peptide biomarker. The term "substances" includes substances that do not directly bind the peptide biomarker and directly modulate a function, but instead indirectly modulate a function of the peptide biomarker. Ligands are also included in the term substances; ligands of the invention (e.g. a natural or synthetic chemical compound, peptide, aptamer, oligonucleotide, antibody or antibody fragment) are capable of binding, preferably specific binding, to the peptide.

The invention further provides a substance according to the invention for use in the treatment of schizophrenia, or predisposition thereto.

Also provided is the use of a substance according to the invention in the treatment of schizophrenia, or predisposition thereto.

Also provided is the use of a substance according to the invention as a medicament.

Yet further provided is the use of a substance according to the invention in the manufacture of a medicament for the treatment of schizophrenia, or predisposition thereto.

A kit for diagnosing or monitoring schizophrenia, or predisposition thereto is provided. Suitably a kit according to the invention may contain one or more components selected from the group: a ligand specific for the peptide biomarker or a structural/shape mimic of the peptide biomarker, one or more controls, one or more reagents and one or more consumables; optionally together with instructions for use of the kit.

The identification of biomarkers for schizophrenia or other psychotic disorders permits integration of diagnostic procedures and therapeutic regimes. Currently there are significant delays in determining effective treatment and hitherto it has not been possible to perform rapid assessment of drug response. Traditionally, many anti-depressant therapies have required treatment trials lasting weeks to months for a given therapeutic approach. Detection of a peptide biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized brain therapies, as assessment of response can be used to fine-tune dosage, minimise the number of prescribed medications, reduce the delay in attaining effective therapy and avoid adverse drug reactions. Thus by monitoring a biomarker of the invention, patient care can be tailored precisely to match the needs determined by the disorder and the pharmacogenomic profile of the patient, the biomarker can thus be used to titrate the optimal dose, predict a positive therapeutic response and identify those patients at high risk of severe side effects.

Biomarker-based tests provide a first line assessment of 'new' patients, and provide objective measures for accurate and rapid diagnosis, in a time frame and with precision, not achievable using the current subjective measures.

Furthermore, diagnostic biomarker tests are useful to identify family members or patients at high risk of developing schizophrenia or other psychotic disorders. This permits initiation of appropriate therapy, or preventive measures, e.g. managing risk factors. These approaches are recognised to improve outcome and may prevent overt onset of the disorder.

Biomarker monitoring methods, biosensors and kits are also vital as patient monitoring tools, to enable the physician to determine whether relapse is due to worsening of the disorder, poor patient compliance or substance abuse. If pharmacological treatment is assessed to be inadequate, then therapy can be reinstated or increased; a change in therapy can be given if appropriate. As the biomarkers are sensitive to the state of the disorder, they provide an indication of the impact of drug therapy or of substance abuse.

In particular embodiments of any of the previously mentioned aspects of the invention, the invention may additionally comprise the use of one or more peptides selected from Coagulation factor 13, Apolipoprotein C1, Apolipoprotein D, Serine/threonine-protein kinase, Mitochondrial ATP-binding cassette sub-family B member 6, E3 ubiquitin-protein ligase, Haptoglobin, ATPase family AAA domain-containing protein 2, Spectrin beta chain erythrocyte, Cartilage oligomeric matrix protein, Mannose-binding protein C, Integrin beta-3, Microtubule-associated protein 2, Cathepsin L2, Complement factor B, Complement component 5, Vitronectin; S-protein, Clusterin (ApoJ), Hyaluronan-binding protein 2, DNA topoisomerase 2-alpha.

The following study illustrates the invention.

### Study

Using global proteomic profiling, a range of circulating biomarkers that differentiate first onset, drug naive schizophrenia patients from healthy controls, were identified. The aim of the study was to establish the utility of these markers for 1) the early diagnosis of schizophrenia, 2) the prediction of drug responses with a focus on metabolic side effects, 3) the selection of appropriate drug treatments at first onset and 4) translational medicine focussing on the validation of preclinical models for novel drug discovery and development. In particular, these biomarkers were tested for patient selection and for monitoring their responses to novel therapeutic treatments.

### Materials and Methods

The first set of 67 serum samples investigated comprised 22 samples taken from first onset drug naïve Schizophrenia patients, 33 samples taken from healthy volunteers and 12 quality control samples. They were prepared randomly and blindly. The second set of 46 serum samples comprised 20 samples taken from first onset drug naïve Schizophrenia patients, 18 samples taken from healthy volunteers and 8 quality control samples. They were prepared randomly and blindly.

Each sample was depleted of the 20 most abundant proteins using immunoaffinity chromatography (ProteoPrep20, Sigma, St. Louis MO), loading a total of 560 µg of protein of each sample onto the depletion column. Buffer exchange was performed with 50 mM ammonium bicarbonate using spin columns (Millipore, Bedford MA) with a 5 kDa molecular weight cut-off. The proteins were reduced using 5 mM dithriotheitol (Sigma, St. Louis MO) at 60 °C for 30 min, and alkylated with 10 mM iodoacetamide (Sigma, St. Louis MO) in the dark at room temperature for 30 min. The proteins were digested using Trypsin (Promega, Madison, WI), at a ratio of 1:50 (w/w Trypsin/Protein) for 16 hours at 37 °C. Digestions were stopped by adding 2.3 µl of 8.8 M HCl to each sample. The samples were stored at -80°C.

Each sample was injected and analysed 3 times followed by a blank injection (to ensure there is no carry-over of peptides from one sample to the other in this sequential process). For each sample, 8 µl were loaded using split-less nano Ultra Performance Liquid Chromatography (10 kpsi nanoAcquity, Waters, Milford MA). Buffers used were A: H₂O+0.1% formic acid; B: Acetonitrile+0.1% formic acid. Desalting of the samples was performed online with 100% buffer A for 3 minutes, using an online Reverse-Phase C18 trapping column (180 µm i.d., 20 mm length and 5 µm particle size) (Waters, Milford MA). The samples were separated using a C18 nanocolumn (75 µm i.d., 200 mm length, 1.7 µm particle size) (Waters, Milford MA), at 300 nl/min.

The nanoUPLC was coupled online through a nanoESI emitter of 7 cm length and 10 µm tip (New Objective, Woburn, MA) to a Quadrupole Time-of-Flight Mass Spectrometer (Qtof Premier, Waters, Milford MA). Data were acquired in MS^{E} (Expression) mode. In this mode, the quadrupole is set to transfer all ions while the collision cell switches from low to high collision energy intermittently throughout the acquisition time. In the low energy scans, collision energy was set to 4 eV, while in the high energy scans it was ramped from 20 to 43 eV. This mode enables accurate mass measurement of both intact peptides as well as fragments, and conservation of the chromatographic profile for both intact peptides and fragments. Mass accuracy was maintained throughout the analysis by the use of a LockSpray apparatus. A reference compound (Glu-Fibrinopeptide B, Sigma, St. Louis MO) was continuously infused using the LockSpray and scanned intermittently every 30 seconds. During data processing, the analyte spectra were corrected based on the difference between the detected m/z peak and the theoretical m/z peak (785.8426 [m+2H]+) of Glu-Fibrinopeptide B.

### Data Processing and Protein Identification

Raw data, acquired in continuum format, were processed using the ProteinLynx Global Server software version 2.3 (also known as Identity^{E}) (Waters, Milford MA). Both quantitative and qualitative information were produced automatically by the software, using the default parameters.

### Quantitative Information

Intensity measurements were obtained by integration of the total ion volume of each extracted, charge-state-reduced, deisotoped and mass corrected ion across the mass spectrometric and chromatographic volume (a 3-dimensional version of extracted ion chromatogram method). In this type of acquisition, chromatographic profile is maintained reproducibly throughout the sample set, thus it is possible to directly compare the intensities of precursor ions across all injections of all samples, following normalization based on the internal standard (digested saccharomyces cerevisiae Enolase) that was added to each sample.

The data set was then filtered using the free software package R (www.r-project.org) and only peptides that were detected in at least 2 out of 3 injections of each sample and at least 70% of the samples were included in the analysis. Those peptides that did not pass these filtering criteria were excluded from the analysis as the quantitative information they generate is of low confidence due to poor replication.

The intensities of the correlating peptides of each identified protein were averaged to produce total protein intensity across all samples in which they were detected, as described by Schwarz *et al.* 2007.

A two-tailed Student's T-Test was performed based on the protein intensities with a significance cut-off of P<0.05. Determined p-values were corrected for multiple hypotheses testing according to Benjamini and Hochberg, significance of Q<0.05.

### Results

| **Protein Name** | **Accession** | **T-test (p value)** |
|---|---|---|
| Coagulation factor 13 | P05160\|F13B_HUMAN | 0.014 |
| Apolipoprotein C1 | P02654\|APOC1_HUMAN | 0.044 |
| Apolipoprotein D | P05090\|APOD_HUMAN | 0.024 |
| Importin 9 | Q96P70\|IPO9_HUMAN | 0.0066 |
| Serine/threonine-protein kinase | Q9NRM7\|LATS2_HUMAN | 0.033 |
| Mitochondrial ATP-binding cassette sub-family B member 6 | Q9NP58\|ABCB6_HUMAN | 0.035 |
| E3 ubiquitin-protein ligase | Q8IWV8\|UBR2_HUMAN | 0.038 |
| NALP8 | Q86W28\|NALP8_HUMAN | 0.0045 |
| Vitamin K-dependent protein S | P07225\|PROS_HUMAN | 0.046 |
| Alpha-1-antichymotrypsin | P01011\|AACT_HUMAN | 0.0064 |
| Lumican | P51884\|LUM_HUMAN | 0.0169 |
| Helicase | Q15477\|SKIV2_HUMAN | 0.019 |
| Haptoglobin | P00738\|HPT_HUMAN | 0.043 |
| Haptoglobin related | P00739\|HPTR_HUMAN | 0.0022 |
| ATPase family AAA domain-containing protein 2 | Q6PL18\|ATAD2_HUMAN | 0.0070 |
| Spectrin beta chain, erythrocyte | P11277\|SPTB1_HUMAN | 0.0084 |
| E3 ubiquitin-protein ligase LRSAM1 | Q6UWE0\|LRSM1_HUMAN | 0.0121 |
| ANKRD26-like family C member 1B | A5A3E0\|A26CB_HUMAN | 0.0124 |
| ATPase family AAA domain-containing protein 2B | Q9ULI0\|ATD2B_HUMAN | 0.0144 |
| Uncharacterized protein C9orf93 | Q6TFL3\|C1093_HUMAN | 0.0177 |
| Cartilage oligomeric matrix protein | P49747\|COMP_HUMAN | 0.0241 |
| Thyroxine-binding globulin | P05543\|THBG_HUMAN | 0.0256 |
| EF-hand domain-containing family member A1 | Q8IYU8\|EFHA1_HUMAN | 0.0264 |
| Mannose-binding protein C | P11226\|MBL2_HUMAN | 0.0289 |
| Alpha-aminoadipic semialdehyde synthase, mitochondrial | Q9UDR5\|AASS_HUMAN | 0.0307 |
| Integrin beta-3 | P051061\|TB3_HUMAN | 0.0318 |
| 1-phosphatidylinositol-4,5-bisphosphate phosphodiesterase gamma-1 | P19174\|PLCG1_HUMAN | 0.0332 |
| Cytosolic carboxypeptidase 2 | Q5U5Z8\|CBPC2_HUMAN | 0.0341 |
| Alpha-1-antichymotrypsin | P01011\|AACT_HUMAN | 0.0344 |
| Microtubule-associated protein 2 | P11137\|MAP2_HUMAN | 0.0349 |
| Uncharacterized protein FLJ36157 | Q8N9V7\|YC009_HUMAN | 0.0388 |
| Cathepsin L2 | 060911\|CATL2_HUMAN | 0.0390 |
| Attractin | 075882\|ATRN_HUMAN | 0.0409 |
| Rootletin | Q5TZA2\|CROCC_HUMAN | 0.0414 |
| Complement component 7 | P10643\|C07_HUMAN | 0.021981 |
| Complement factor B | P00751\|CFAB_HUMAN | 0.027791 |
| Complement component 5 | P01031\|C05_HUMAN | 0.028466 |
| IgMu | P01871\|MUC_HUMAN | 0.031597 |
| Afamin | P43652\|AFAM_HUMAN | 0.038915 |
| Kinesin-like protein | Q9ULI4\|KI26A_HUMAN | 0.0381939 |
| Vitronectin; S-protein | P04004\|VTNC_HUMAN | 0.00111 |
| Clusterin (ApoJ) | P10909\|CLUS_HUMAN | 0.04233 |
| Poly [ADP-ribose] polymerase 1 (PARP1) | P09874\|PARP1_HUMAN | 0.01680 |
| Hyaluronan-binding protein 2 | Q14520\|HABP2_HUMAN | 0.02213 |
| Ig gamma-1 chain C region | P01857\|IGHG1_HUMAN | 0.03141 |
| Serum paraoxonase/arylesterase 1 | P27169\|PON1_HUMAN | 0.04709 |
| Inter-alpha-trypsin inhibitor heavy chain H2 | P19823\|ITIH2_HUMAN | 0.01793 |
| Inter-alpha-trypsin inhibitor heavy chain H4 | Q14624\|ITIH4_HUMAN | 0.01890 |
| Apolipoprotein C-III | P02656\|APOC3_HUMAN | 0.00538 |
| Pregnancy Zone protein | P2G742\|PZP_HUMAN | 0.0445 |
| DNA topoisomerase 2-alpha | P11388\|TOP2A_HUMAN | 0.0241 |
| Heparin cofactor 2 | P05546\|HEP2_HUMAN | 0.0424 |
| Complement C1 r subcomponent | P00736\|C1R_HUMAN | 0.0489 |

## Claims

1. The *in vitro* use of Importin 9, as a biomarker for schizophrenia, or predisposition thereto.

2. The *in vitro* use according to claim 1, additionally comprising the use of one or more additional peptides selected from, NALP8, Vitamin K-dependent protein S, Alpha-1-antichymotrypsin, Lumican, Helicase, Haptoglobin related, ANKRD26-like family C member 1 B, ATPase family AAA domain-containing protein 2B, Uncharacterized protein C9orf93, Thyroxine-binding globulin, EF-hand domain-containing family member A1, Alpha-aminoadipic semialdehyde synthase, mitochondrial, 1-phosphatidylinositol-4,5-bisphosphate phosphodiesterase gamma-1, Cytosolic carboxypeptidase 2, Uncharacterized protein FLJ36157, Attractin, Rootletin, Complement component 7, Afamin, Kinesin-like protein, Poly [ADP-ribose] polymerase 1 (PARP1), Ig gamma-1 chain C region, Serum paraoxonase/arylesterase 1, Inter-alpha-trypsin inhibitor heavy chain H2, Inter-alpha-trypsin inhibitor heavy chain H4, Apolipoprotein C-III, Pregnancy Zone protein, Heparin cofactor 2 and Complement C1r subcomponent, as a biomarker for schizophrenia, or predisposition thereto.

3. The *in vitro* use according to claim 1 or claim 2, additionally comprising the use of one or more additional peptides selected from E3 ubiquitin-protein ligase LRSAM1, IgMu, Coagulation factor 13, Apolipoprotein C1, Apolipoprotein D, Serine/threonine-protein kinase, Mitochondrial ATP-binding cassette sub-family B member 6, E3 ubiquitin-protein ligase, Haptoglobin, ATPase family AAA domain-containing protein 2, Spectrin beta chain erythrocyte, Cartilage oligomeric matrix protein, Mannose-binding protein C, Integrin beta-3, Microtubule-associated protein 2, Cathepsin L2, Complement factor B, Complement component 5, Vitronectin; S-protein, Clusterin (ApoJ), Hyaluronan-binding protein 2 and DNA topoisomerase 2-alpha, as a biomarker for schizophrenia, or predisposition thereto.

4. A method of diagnosing or monitoring schizophrenia, or predisposition thereto, comprising detecting and/or quantifying, in a sample from a test subject, the biomarker listed in claim 1.

5. A method according to claim 4, additionally comprising detecting and/or quantifying, in a sample from a test subject, one or more of the additional peptide biomarkers listed in claim 2 or claim 3.

6. A method of monitoring efficacy of a therapy in a subject having, suspected of having, or of being predisposed to schizophrenia, comprising detecting and/or quantifying, in a sample from said subject, the biomarker listed in claim 1.

7. A method according to claim 6, additionally comprising detecting and/or quantifying, in a sample from said subject, one or more of the additional peptide biomarkers listed in claim 2 or claim 3.

8. A method according to any of claims 4 to 7, which is conducted on samples taken on two or more occasions from a test subject.

9. A method according to any of claims 4 to 8, further comprising comparing the level of the biomarker present in samples taken on two or more occasions.

10. A method according to any of claims 4 to 9, comprising comparing the amount of the biomarker in said test sample with the amount present in one or more samples taken from said subject prior to commencement of therapy, and/or one or more samples taken from said subject at an earlier stage of therapy.

11. A method according to any of claims 4 to 10, further comprising detecting a change in the amount of the biomarker in samples taken on two or more occasions.

12. A method according to any of claims 4 to 11, comprising comparing the amount of the biomarker present in said test sample with one or more controls.

13. A method according to any of claims 4 to 12, wherein samples are taken prior to and/or during and/or following therapy for schizophrenia.

14. A method according to any of claims 4 to 13, wherein the biological sample is cerebrospinal fluid, whole blood, blood serum, plasma, urine, saliva, or other bodily fluid, or breath, condensed breath, or an extract or purification therefrom, or dilution thereof.

15. Use of a kit for monitoring or diagnosing schizophrenia, wherein said kit comprises a biosensor which detects and/or quantifies the biomarker listed in claim 1, wherein said biosensor comprises a ligand capable of specific binding to the biomarker listed in claim 1.

## Patentansprüche

1. *In-vitro*-Verwendung von Importin 9 als Biomarker für Schizophrenie oder die Prädisposition dafür.

2. *In-vitro*-Verwendung nach Anspruch 1, die zusätzlich die Verwendung von einem oder mehreren zusätzlichen Peptiden beinhaltet, die ausgewählt sind aus NALP8, Vitamin K-abhängigem Protein S, Alpha-1-antichymotrypsin, Lumican, Helicase, Haptoglobin-bezogenes Protein, Mitglied 1 B der ANKRD26-ähnlichen Familie C, Protein 2B, das ATPase-Familie AAA-Domäne enthält, uncharakterisiertes Protein C9orf93, Thyroxin-Bindungsglobulin, EF-Hand-Domäne-enthaltendes Familienmitglied A1, mitochondriale Alpha-aminoadipische Semialdehydsynthase, 1-Phosphatidylinositol-4,5-bisphosphatphosphodiesterase Gamma-1, cytosolische Carboxypeptidase 2, uncharakterisiertes Protein FLJ36157, Attractin, Rootletin, Complement-Komponente 7, Afamin, kinesinähnliches Protein, Poly[ADP-ribose]polymerase 1 (PARP1), Ig Gamma-1-Ketten-C-Region, Serumparaoxonase/Arylesterase 1, schwere Inter-alpha-trypsin-Inhibitorkette H2, schwere Inter-alpha-trypsin-Inhibitorkette H4, Apolipoprotein C-III, Schwangerschaftszonenprotein, Heparin-Cofactor 2 und Complement C1r r Subkomponente, als Biomarker für Schizophrenie oder die Prädisposition dafür.

3. *In-vitro*-Verwendung nach Anspruch 1 oder Anspruch 2, die zusätzlich die Verwendung von einem oder mehreren zusätzlichen Peptiden beinhaltet, die ausgewählt sind aus E3 Ubiquitin-Proteinligase LRSAM1, IgMu, Koagulationsfaktor 13, Apolipoprotein C1, Apolipoprotein D, Serin/Threonin-Protein-Kinase, Mitglied 6 der mitochondrialen ATP-bindenden Kassetten-Subfamilie B, E3 Ubiquitin-Protein-Ligase, Haptoglobin, Protein 2, das ATPase-Familie AAA-Domäne enthält, Spectrin-Beta-Kette-Erythrocyt, oligomeres Knorpelmatrixprotein, mannosebindendes Protein C, Integrin Beta-3, Mikrotubul-assoziiertes Protein 2, Cathepsin L2, Complement Faktor B, Complement Komponente 5, Vitronectin; S-Protein, Clusterin (ApoJ), Hyaluronanbindendes Protein 2 und DNA-Topoisomerase-2-alpha, als Biomarker für Schizophrenie oder die Prädisposition dafür.

4. Verfahren zum Diagnostizieren oder Überwachen von Schizophrenie oder der Prädisposition dafür, das das Erkennen und/oder Quantifizieren, in einer Probe von einer Testperson, des in Anspruch 1 aufgeführten Biomarkers beinhaltet.

5. Verfahren nach Anspruch 4, das zusätzlich das Erkennen und/oder Quantifizieren, in einer Probe von einer Testperson, von einem oder mehreren der in Anspruch 2 oder Anspruch 3 aufgeführten zusätzlichen Peptidbiomarker beinhaltet.

6. Verfahren zum Überwachen der Wirksamkeit einer Therapie in einer Person, die Schizophrenie hat, bei der Schizophrenie vermutet wird oder die dafür prädisponiert ist, das das Erkennen und/oder Quantifizieren, in einer Probe von der genannten Person, des in Anspruch 1 aufgeführten Biomarkers beinhaltet.

7. Verfahren nach Anspruch 6, das zusätzlich das Erkennen und/oder Quantifizieren, in einer Probe von der genannten Person, von einem oder mehreren der in Anspruch 2 oder Anspruch 3 zusätzlichen aufgeführten Peptidbiomarker beinhaltet.

8. Verfahren nach einem der Ansprüche 4 bis 7, das an Proben durchgeführt wird, die zwei oder mehrere Male von einer Testperson genommen werden.

9. Verfahren nach einem der Ansprüche 4 bis 8, das ferner das Vergleichen des Spiegels des Biomarkers beinhaltet, der in zwei oder mehrere Male genommenen Proben vorhanden ist.

10. Verfahren nach einem der Ansprüche 4 bis 9, das das Vergleichen der Menge des Biomarkers in der genannten Testprobe mit der Menge beinhaltet, die in einer oder mehreren von der genannten Person vor Therapiebeginn genommenen Proben und/oder einer oder mehreren von der genannten Person in einer früheren Therapiephase genommenen Proben vorhanden ist.

11. Verfahren nach einem der Ansprüche 4 bis 10, das ferner das Erkennen einer Änderung der Menge des Biomarkers in zwei oder mehrere Male genommenen Proben beinhaltet.

12. Verfahren nach einem der Ansprüche 4 bis 11, das das Vergleichen der Menge des in der genannten Testprobe vorhandenen Biomarkers mit einer oder mehreren Kontrollen beinhaltet.

13. Verfahren nach einem der Ansprüche 4 bis 12, wobei die Proben vor und/oder während und/oder nach einer Schizophrenietherapie genommen werden.

14. Verfahren nach einem der Ansprüche 4 bis 13, wobei die biologische Probe Rückenmarksflüssigkeit, Vollblut, Blutserum, Plasma, Urin, Speichel oder anderes Körperfluid, oder Atem, kondensierter Atem, oder ein(e) Extrakt oder Reinigung davon, oder eine Verdünnung davon ist.

15. Verwendung eines Kits zum Überwachen oder Diagnostizieren von Schizophrenie, wobei der genannte Kit einen Biosensor umfasst, der den in Anspruch 1 aufgeführten Biomarker erkennt und/oder quantifiziert, wobei der genannte Biosensor einen Liganden umfasst, der sich spezifisch an den in Anspruch 1 aufgeführten Biomarker binden kann.

## Revendications

1. Utilisation *in vitro* de l'importine 9 comme biomarqueur de la schizophrénie ou d'une prédisposition à cette maladie.

2. Utilisation *in vitro* selon la revendication 1, qui comprend également l'utilisation d'un ou de plusieurs peptides additionnels sélectionnés parmi les suivants : NALP8, protéine S dépendante de la vitamine K, alpha-1-antichymotrypsine, lumicane, hélicase, protéine apparentée à l'haptoglobine, membre 1 B de la famille C des protéines codées par des gènes de type ANKRD26, protéine 2B à domaine AAA de la famille des ATPases, protéine non caractérisée codée par C9orf93, globuline liant la thyroxine, membre A1 de la famille des protéines contenant un domaine à motif main EF, semialdéhyde-synthase alpha-aminoadipique mitochondriale, 1-phosphatidylinositol-4,5-bisphosphate phosphodiestérase gamma-1, carboxypeptidase 2 cytosolique, protéine FLJ36157 non caractérisée, attractine, rootletine, composant 7 du complément, afamine, protéine de la famille des kinésines, poly[ADP-ribose] polymérase 1 (PARP1), région de la chaîne C de l'Ig gamma-1, paraoxonase/arylestérase 1 sérique, chaîne lourde H2 de l'inter-alpha trypsine inhibiteur, chaîne lourde H4 de l'inter-alpha trypsine inhibiteur, apolipoprotéine C-III, protéine zone grossesse, cofacteur2 de l'héparine et sous-composant C1r du complément, comme biomarqueur de la schizophrénie ou d'une prédisposition à cette maladie.

3. Utilisation *in vitro* selon la revendication 1 ou la revendication 2, qui comprend en outre l'utilisation d'un ou de plusieurs peptides additionnels sélectionnés parmi les suivants : ligase E3 ubiquitine-protéine LRSAM1, IgMu, facteur de coagulation 13, apolipoprotéine C1, apolipoprotéine D, protéine sérine/thréonine kinase, membre 6 de la sous-famille B des transporteurs à boîtes de liaison à l'ATP mitochondriaux, ligase E3 ubiquitine-protéine, haptoglobine, protéine 2 à domaine AAA de la famille des ATPases, chaîne bêta de la spectrine érythrocytaire, protéine matricielle oligomère du cartilage, protéine C liant le mannose, intégrine bêta-3, protéine 2 associée aux microtubules, cathepsine L2, facteur B du complément, composant 5 du complément, vitronectine, protéine S, clusterine (ApoJ), protéine 2 liant l'hyaluronane et ADN-topoisomérase 2-alpha, comme biomarqueur de la schizophrénie ou d'une prédisposition à cette maladie.

4. Méthode de diagnostic ou de suivi de la schizophrénie ou d'une prédisposition à cette maladie, qui comprend la détection et/ou la quantification du biomarqueur mentionné à la revendication 1 dans un échantillon obtenu chez un sujet testé.

5. Méthode selon la revendication 4, qui comprend également la détection et/ou la quantification d'un ou de plusieurs des biomarqueurs peptidiques additionnels mentionnés à la revendication 2 ou à la revendication 3 dans un échantillon obtenu chez un sujet testé.

6. Méthode de suivi de l'efficacité d'une thérapie chez un sujet qui souffre ou qui est suspecté de souffrir de schizophrénie ou d'une prédisposition à cette maladie, qui comprend la détection et/ou la quantification du biomarqueur mentionné à la revendication 1 dans un échantillon obtenu chez ledit sujet.

7. Méthode selon la revendication 6, qui comprend également la détection et/ou la quantification d'un ou de plusieurs des biomarqueurs peptidiques additionnels mentionnés à la revendication 2 ou à la revendication 3 dans un échantillon obtenu chez ledit sujet.

8. Méthode selon l'une quelconque des revendications 4 à 7, qui est effectuée sur des échantillons obtenus à deux occasions ou plus chez un sujet testé.

9. Méthode selon l'une quelconque des revendications 4 à 8, qui comprend également la comparaison des taux du biomarqueur présents dans les échantillons obtenus à deux occasions ou plus.

10. Méthode selon l'une quelconque des revendications 4 à 9, qui comprend la comparaison de la quantité de biomarqueur présente dans ledit échantillon testé à la quantité présente dans un ou plusieurs échantillons obtenus à deux occasions ou plus chez ledit sujet avant l'instauration d'une thérapie et/ou dans un ou plusieurs échantillons obtenus chez ledit sujet à un stade plus précoce de la thérapie.

11. Méthode selon l'une quelconque des revendications 4 à 10, qui comprend également la détection d'un changement de la quantité du biomarqueur dans les échantillons obtenus à deux occasions ou plus.

12. Méthode selon l'une quelconque des revendications 4 à 11, qui comprend la comparaison de la quantité de biomarqueur présente dans ledit échantillon testé à celle présente dans un ou plusieurs contrôles.

13. Méthode selon l'une quelconque des revendications 4 à 12, dans laquelle les échantillons sont obtenus avant et/ou pendant et/ou après une thérapie contre la schizophrénie.

14. Méthode selon l'une quelconque des revendications 4 à 13, dans laquelle l'échantillon biologique est le liquide céphalorachidien, le sang entier, le sérum sanguin, le plasma, l'urine, la salive ou tout autre fluide corporel, l'haleine, l'haleine condensée ou un extrait ou produit de purification ou dilution de ceux-ci.

15. Utilisation d'un kit pour le suivi ou le diagnostic de la schizophrénie, dans laquelle ledit kit comprend un biocapteur qui détecte et/ou quantifie le biomarqueur mentionné à la revendication 1, ledit biocapteur comprenant un ligand capable de se lier spécifiquement au biomarqueur mentionné à la revendication 1.
